# EUROPÄISCHE PATENTSCHRIFT

(11) **EP 1 578 483 B1**
(45) Veröffentlichungstag und Bekanntmachung des Hinweises auf die Patenterteilung: **13.07.2011**
(21) Anmeldenummer: 03782289.7
(22) Anmeldetag: 03.12.2003
(51) Int. Cl.: A61N 1/04

(54) **ELEKTRODENPACKUNG**
ELECTRODE PACK
EMBALLAGE D'ELECTRODES

(30) Priorität: 20.12.2002 DE 10261601
(43) Veröffentlichungstag der Anmeldung: 28.09.2005
(73) Patentinhaber: Metrax GmbH, D-78628 Rottweil (DE)
(72) Erfinder: BUCHER, Jürgen, 78628 Rottweil (DE)
(74) Vertreter: Fleck, Hermann-Josef
(86) Internationale Anmeldenummer: PCT/EP2003/013610
(87) Internationale Veröffentlichungsnummer: WO 2004/058345

(56) Entgegenhaltungen:
- US-A- 5 402 884
- US-A- 5 579 919
- US-A- 5 984 102
- US-A- 6 048 640
- US-A- 6 101 413

## Beschreibung

Die Erfindung bezieht sich auf eine Elektrodenpackung mit mindestens zwei an einen Patienten anzulegenden flächigen Elektroden, die mit einem Gel versehene flächige Kontaktbereiche auf jedem Elektrodenkörper sowie Anschlusskabel an jedem Elektrodenkörper aufweisen und mit luftdichten Abschlussmitteln versehen sind.

Derartige Elektroden werden insbesondere in Verbindung mit Defibrillatoren verwendet und müssen stets funktionsfähig sein, vor allem wenn sie bei automatischen externen Defibrillatoren (AEDs) stets schnell betriebsbereit sein sollen. Die Elektrodenpackung soll garantieren, dass die Kontaktbereiche möglichst lange in einem funktionsfähigen Zustand gehalten werden. Eine derartige Elektrodenpackung ist in der US 5,402,884 und ähnlich auch in den US 5,579,919 und US 6,048,640 angegeben. Dabei schließt die Verpackung die beiden Elektroden luftdicht ein, wobei auch das Kabel unter luftdichter Abdichtung durch die Verpackung geführt ist.

Der Erfindung liegt die Aufgabe zugrunde, eine Elektrodenpackung der eingangs genannten Art bereit zu stellen, mit der eine einfache Handhabung ermöglicht wird und Packungsmaterial eingespart werden kann.

Diese Aufgabe wird mit den Merkmalen des Anspruches 1 gelöst. Hiernach ist vorgesehen, dass die Abschlussmittel die Kontaktbereiche umgebende, an den beiden Elektrodenkörpern luftdicht in Verbindung gebrachte Dichtmittel aufweisen, wobei die Anschlusskabel außerhalb der Dichtmittel aus den Elektrodenkörpern herausgeführt sind.

Mit diesen Maßnahmen können die beiden Elektroden einfach auseinander genommen und von der Verpackung getrennt werden, wobei die Anschlusskabel nicht stören und nicht gesondert von dem Packungsmaterial getrennt werden müssen. Auch kann bei dieser Konstruktion leicht Packungsmaterial eingespart werden.

Eine für die Handhabung und den Aufbau günstige Ausbildung besteht darin, dass die Dichtmittel eine umlaufende Dichtringanordnung aufweisen, die über Verbindungsmittel luftdicht mit beiden Elektrodenkörpern abziehbar verbunden ist.

Hierbei besteht eine für die Anordnung und Handhabung vorteilhafte Ausgestaltung darin, dass die Dichtringanordnung auf den einander zugekehrten, die Kontaktbereiche tragenden Seiten der Elektrodenkörper jeweils mindestens eine angeformte Dichtwulst aufweist, die bezüglich der beiden Elektrodenkörper miteinander zur Deckung gebracht und mit dem Verbindungsmittel miteinander verbunden sind oder dass zwischen den einander zugekehrten Seiten der beiden Elektrodenkörper mindestens ein um die Kontaktbereiche umlaufender Dichtring angeordnet ist, der auf seinen beiden, den Elektrodenkörpern zugekehrten Seiten jeweils über Verbindungsmittel mit den Elektrodenkörpern verbunden ist. Auf diese Weise werden die Elektroden auch in definierter Gegenüberlage gehalten.

Ist vorgesehen, dass zwischen den einander zugekehrten Kontaktbereichen mindestens eine Zwischenschicht angeordnet ist, so sind die Kontaktbereiche zusätzlich geschützt.

Eine weitere vorteilhafte Ausgestaltung für die Handhabung und den Aufbau besteht darin, dass die Dichtringanordnung Teil einer den Kontaktbereich abdeckenden Folie ist, die zwischen den einander zugekehrten Kontaktbereichen mindestens eine isolierende Zwischenschicht bildet, wobei ein jeweiliger Teil über ein jeweiliges Verbindungsmittel mit einem ihm zugeordneten Elektrodenkörper verbunden ist. Beim Auseinandernehmen der Elektroden kann das Verpackungsmaterial leicht entfernt werden.

Die Handhabung wird weiterhin dadurch begünstigt, dass mindestens eine der beiden Elektroden mit einer Grifflasche zum Auseinanderziehen der beiden Elektroden versehen ist.

Hierbei besteht eine vorteilhafte Ausbildung darin, dass die mindestens eine Grifflasche an die Dichtringanordnung oder die mindestens eine Zwischenschicht angebunden ist.

Der funktionsfähige Zustand der Elektroden wird weiterhin dadurch zuverlässig sichergestellt, dass die Zwischenschicht als Wirkmittel für einen elektrischen Elektrodentest eingebunden ist.

Die Erfindung wird nachfolgend anhand von Ausführungsbeispielen unter Bezugnahme auf die Zeichnungen näher erläutert. Es zeigen:
- Fig. 1A: eine schematische Darstellung einer Elektrodenpackung im Quer- schnitt,
- Fig. 1B: einen Ausschnitt einer Elektrodenpackung nach Fig. 1A mit einer ab- gewandelten Abdichtanordnung,
- Fig. 2: die Elektrodenpackung nach Fig. 1A in Draufsicht und
- Fig. 3: ein weiteres Ausführungsbeispiel einer Elektrodenpackung in seit- licher Ansicht.

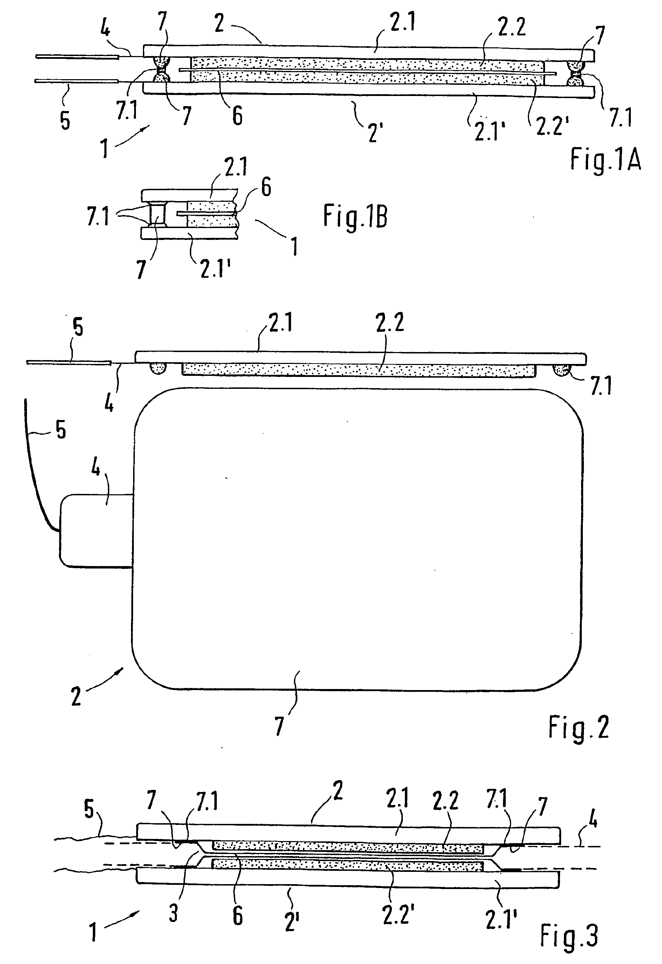

Die Fig. 1A, 1B, 2 und 3 zeigen verschiedene Darstellungen einer Elektrodenpackung 1 mit einer ersten und zweiten Elektrode 2, 2', die jeweils einen Elektrodenkörper 2.1, 2.1' mit auf einer Seite angeordnetem Kontaktbereich 2.2, 2.2' mit einer an einem Patienten anzulegenden Kontaktschicht aufweisen. Die beiden Elektroden 2, 2' sind mit ihren Kontaktbereichen gegeneinander gekehrt und vorzugsweise mittels einer Zwischenschicht 6 voneinander getrennt. An den beiden Elektrodenkörpern 2.1, 2.1' sind jeweils Anschlusskabel 5 zum Verbinden mit einer (nicht gezeigten) Ansteuereinrichtung eines Defibrillators angeschlossen, um den Patienten mit einer stimulierenden elektrischen Spannung zu beaufschlagen und gegebenenfalls auch einen Funktionstest zum Feststellen der Funktionsfähigkeit der Elektroden 2, 2' auch im verpackten Zustand durchführen zu können. Die Kontaktbereiche 2.2, 2.2' sind luftdicht mittels einer Abdichtvorrichtung mit Dichtmitteln 7 und Verbindungsmitteln 7.1 nach außen hin geschlossen, so dass ein Austrocknen der üblicherweise im Kontaktbereich 2.2, 2.2' vorhandenen Gelschicht zu verhindert und die Funktionsfähigkeit des Kontaktbereichs auf lange Sicht gewährleistet ist.

Die Dichtmittel 7 weisen einen luftdicht um den jeweiligen Kontaktbereich 2.2, 2.2' umlaufenden Dichtring auf, wie beispielsweise aus Fig. 2 ersichtlich. Außerhalb des Dichtringes sind die Anschlusskabel 5 aus dem jeweiligen Elektrodenkörper 2.1, 2.1' herausgeführt. Die Dichtmittel 7 können dabei in verschiedener Weise ausgeführt sein, wie die Fig. 1A bis 3 zeigen.

Gemäß Fig. 1A ist an den einander zugekehrten Seiten der Elektroden 2, 2' der Elektrodenpackung 1, auf der sich auch die Kontaktbereiche 2.2, 2.2' befinden, jeweils ein wulstartiger oder lippenartiger Dichtring angeformt, angeklebt oder angeschweißt. Die beiden Dichtringe der jeweiligen Elektroden 2, 2' sind im Packungszustand deckungsgleich und in ihrem einander angrenzenden Bereich miteinander verbunden. Für die Verbindung sind verschiedene Verbindungsmittel 7.1 denkbar, die ein einfaches Auseinanderziehen der beiden Elektroden 2, 2' für den Gebrauch ermöglichen, beispielsweise ein Kleber oder eine Verschweißung oder auch eine Verbindung mit einer Nut/Vorsprung-Anordnung, wobei die Dichtringe aus geeignetem elastischen Dichtmaterial, z.B. Silikon bestehen. Mittels an den Elektrodenkörpern 2.1, 2.1' oder der Dichtmittelanordnung 7 angebrachter Grifflaschen 4, die seitlich über den Rand der Elektroden 2, 2' vorstehen, können die beiden Elektroden leicht gefasst und auseinandergezogen werden. Die Verbindungsmittel 2.1 sind dabei so abgestimmt, dass sie ein einfaches Auseinanderziehen ermöglichen und andererseits einen luftdichten Abschluss gewährleisten. Auch mehrere nebeneinander angeordnete umlaufende Dichtringe können vorgesehen sein.

Die Fig. 1B zeigt gegenüber dem Ausführungsbeispiel nach Fig. 1A eine abgewandelte Abdichtvorrichtung, bei der das umlaufende Dichtmittel 7 als zwischen den einander zugekehrten Seiten der beiden Elektroden 2, 2' angeordnetes Dichtungselement ausgebildet ist, die mit den angrenzenden Seiten der Elektrodenkörper 2.1, 2.1' verklebt oder verschweißt sind. Auch hierbei ist eine Art Nut/Vorsprung-Verbindung wie bei dem erstgenannten Ausführungsbeispiel möglich. Auch hierbei sind die Anschlusskabel 5 außerhalb der Dichtmittel 7 aus dem jeweiligen Elektrodenkörper 2.1, 2.1' herausgeführt. Außerdem sind auch hierbei Grifflaschen 4 in der vorstehend angegebenen Weise vorteilhaft.

Bei dem in Fig. 3 gezeigten weiteren Ausführungsbeispiel sind die Dichtmittel 7 an einer Abdeckfolie der beiden Kontaktbereiche 2.2, 2.2' angebracht und bilden einen z.B. einstückig angeformten oder daran angeschweißten oder angeklebten Teil derselben. Dabei kann die die Zwischenschicht 6 bildende Abdeckfolie einschichtig mit zwei voneinander getrennten Randabschnitten oder zweischichtig mit einem jeweiligen Randabschnitt ausgebildet sein, wobei die beiden Schichten vorteilhaft zum Einhalten der Lage miteinander verbunden sind. Die über die jeweiligen Kontaktbereiche 2.2, 2.2' seitlich hinausragenden Abschnitte sind mit dem jeweiligen Elektrodenkörper 2.1, 2.1' als Dichtmittel 7 mittels Verbindungsmittel 7.1 verbunden. Auch hierbei kann das Verbindungsmittel 7.1 z.B. ein geeigneter Kleber oder eine Schweißverbindung zum luftdichten Abschluss sein. Hinsichtlich der Anbringung der Grifflaschen 4 und der Verbindung mit den Anschlusskabeln 5 ist die Elektrodenpackung 1 vorteilhaft entsprechend den vorgenannten Ausführungsbeispielen ausgebildet. Ist bei zweischichtigem Aufbau der Zwischenschicht 6 aus folienartigem Verpackungsmaterial 3 die Verbindung zwischen den beiden Schichten fester als die Verbindung der Dichtmittel 7 mit den Elektrodenkörpern 2.1, 2.1', wird das Verpackungsmaterial beim Auseinanderziehen der beiden Elektroden 2, 2' von mindestens einem Elektrodenkörper 2.1, 2.1' getrennt und kann leicht von den Elektroden entfernt werden.

Bei allen genannten Ausführungsbeispielen lässt sich die Zwischenschicht 6 vorteilhaft z.B. als Dielektrikum verwenden, um von Zeit zu Zeit einen automatischen Elektrodentest durchzuführen und die Funktionssicherheit der Elektroden 2, 2' zu registrieren und zu gewährleisten.

## Patentansprüche

1. Elektrodenpackung mit mindestens zwei an einen Patienten anzulegenden flächigen Elektroden (2, 2'), die mit einem Gel versehene flächige Kontaktbereiche (2.2, 2.2') auf jedem Elektrodenkörper (2.1, 2.1') sowie Anschlusskabel (5) an jeden Elektrodenkörper aufweisen und mit luftdichten Abschlussmitteln (3, 6, 7) versehen sind,
**dadurch gekennzeichnet,**
**dass** die Abschlussmittel (3, 6, 7) die Kontaktbereiche (2.2, 2.2') umgebende, an den beiden Elektrodenkörpern (2.1, 2.1') luftdicht in Verbindung gebrachte Dichtmittel (7, 7.1; 6.1) aufweisen, wobei die Anschlusskabel außerhalb der Dichtmittel (7, 7.1) aus den Elektrodenkörpern (2.1, 2.1') herausgeführt sind.

2. Elektrodenpackung nach Anspruch 1,
**dadurch gekennzeichnet,**
**dass** die Dichtmittel (7, 7.1) eine umlaufende Dichtringanordnung (7) aufweisen, die über Verbindungsmittel (7.1) luftdicht mit beiden Elektrodenkörpern (2.1, 2.1') abziehbar verbunden ist.

3. Elektrodenpackung nach Anspruch 2,
**dadurch gekennzeichnet,**
**dass** die Dichtringanordnung (7) auf den einander zugekehrten, die Kontaktbereiche (2.2, 2.2') tragenden Seiten der Elektrodenkörper (2.1, 2.1') jeweils mindestens eine angeformte Dichtwulst (Fig. 1 A) aufweist, die bezüglich der beiden Elektrodenkörper (2.1, 2.1') miteinander zur Deckung gebracht und mit dem Verbindungsmittel (7.1) miteinander verbunden sind oder
**dass** zwischen den einander zugekehrten Seiten der beiden Elektrodenkörper (2.1, 2.1') mindestens ein um die Kontaktbereiche (2.2, 2.2') umlaufender Dichtring angeordnet ist, der auf seinen beiden, den Elektrodenkörpern (2, 2') zugekehrten Seiten jeweils über Verbindungsmittel (7.1) mit den Elektrodenkörpern (2, 2') verbunden ist.

4. Elektrodenpackung nach Anspruch 2 oder 3,
**dadurch gekennzeichnet,**
**dass** zwischen den einander zugekehrten Kontaktbereichen (2.2, 2.2') mindestens eine Zwischenschicht (6) angeordnet ist.

5. Elektrodenpackung nach Anspruch 1 oder 2,
**dadurch gekennzeichnet,**
**dass** die Dichtringanordnung (7) Teil einer den Kontaktbereich (2.2, 2.2') abdeckenden Folie ist, die zwischen den einander zugekehrten Kontaktbereichen (2.2, 2.2') mindestens eine isolierende Zwischenschicht (6) bildet, wobei ein jeweiliger Teil über ein jeweiliges Verbindungsmittel (7.1) mit einem ihm zugeordneten Elektrodenkörper (2.1, 2.1') verbunden ist.

6. Elektrodenpackung nach einem der vorhergehenden Ansprüche,
**dadurch gekennzeichnet,**
**dass** mindestens eine der beiden Elektroden (2, 2') mit einer Grifflasche (4) zum Auseinanderziehen der beiden Elektroden (2, 2') versehen ist.

7. Elektrodenpackung nach Anspruch 6,
**dadurch gekennzeichnet,**
**dass** die mindestens eine Grifflasche (4) an die Dichtringanordnung (7) oder die mindestens eine Zwischenschicht (6) angebunden ist.

8. Elektrodenpackung nach einem der Ansprüche 4 bis 7,
**dadurch gekennzeichnet,**
**dass** die Zwischenschicht (6) als Wirkmittel für einen elektrischen Elektrodentest eingebunden ist.

## Claims

1. Electrode pack comprising at least two flat electrodes (2, 2'), said electrodes are to be placed on a patient and have flat contact areas (2.2, 2.2'), which are located on each electrode body (2.1, 2.1') and which are provided with a gel, have a connecting cable (5) on each electrode body and are provided with air-tight closing means (3, 6, 7),
**characterised in that**,
the closing means (3, 6, 7), while encircling the contact areas (2.2, 2.2'), have sealing means (7, 7.1; 6.1) that are joined in an air-tight manner to both electrode bodies (2.1, 2.1'), and the connecting cables are guided out of the electrode bodies (2.1, 2.1) outside of the sealing means (7, 7.1).

2. Electrode pack according to claim 1,
**characterised in**
**that** the sealing means (7, 7.1) have a revolving sealing ring arrangement (7) which, via connecting means (7.1), is removably connected with both electrode bodies (2.1, 2.1') in an air-tight manner.

3. Electrode pack according to claim 2,
**characterised in that**,
the sealing ring arrangement (7) on the mutually facing sides of the electrode bodies (2.1, 2.1'), which carry the contact areas (2.2, 2.2'), always has at least one integrally formed sealing bead (Fig. 1A), the sides in respect of both electrode bodies (2.1, 2.1') being matched and interlinked with the connecting means (7.1) or that, between the mutually facing sides of both electrode bodies (2.1, 2.1'), at least one sealing ring revolving around the contact areas (2.2, 2.2') is arranged, with the sealing ring always connected on both its sides facing the electrode bodies (2, 2'), via connecting means (7.1), with the electrode bodies (2, 2').

4. Electrode pack according to claim 2 or 3,
**characterised in that**,
at least one intermediate layer (6) is arranged between the contact areas (2.2, 2.2') facing one another.

5. Electrode pack according to claim 1 or 2,
**characterised in that**,
the sealing ring arrangement (7) is part of a foil covering the contact area (2.2, 2.2') which, between the contact areas facing one another (2.2, 2.2'), forms at least one isolating intermediate layer (6), with a respective part connected via a respective connecting means (7.1) with an electrode body (2.1, 2.1') allocated to the respective part.

6. Electrode pack according to one of the preceding claims,
**characterised in that**, at least one of the two electrodes (2, 2') is provided with a gripping tab (4) for pulling the two electrodes (2, 2') apart.

7. Electrode pack according to claim 6,
**characterised in that**,
the at least one gripping tab (4) is linked to the sealing ring arrangement (7) or the at least one intermediate layer (6).

8. Electrode pack according to one of the claims 4 to 7,
**characterised in that**,
the intermediate layer (6) is integrated as an effecting means for an electrical electrode test.

## Revendications

1. Paquet d'électrodes comportant au moins deux électrodes plates (2, 2') à poser sur un patient, qui présentent des zones de contact plates (2.2, 2.2') munies d'un gel sur chaque corps d'électrode (2.1, 2.1') ainsi qu'un câble de raccordement (5) sur chaque corps d'électrode et qui sont munies de moyens de fermeture (3, 6, 7) étanches à l'air,
**caractérisé en ce**
**que** les moyens de fermeture (3, 6, 7) présentent des moyens d'étanchéité (7, 7.1 ; 6.1) entourant les zones de contact (2.2, 2.2'), mis en contact de manière étanche à l'air sur les deux corps d'électrode (2.1, 2.1'), les câbles de raccordement étant menés hors des corps d'électrode (2.1, 2.1') à l'extérieur des moyens d'étanchéité (7, 7.1).

2. Paquet d'électrodes selon la revendication 1,
**caractérisé en ce**
**que** les moyens d'étanchéité (7, 7.1) présentent un agencement de bagues d'étanchéité (7) périphérique qui est relié par des moyens de liaison (7.1) de manière étanche à l'air et décollable aux deux corps d'électrodes (2.1, 2.1').

3. Paquet d'électrodes selon la revendication 2,
**caractérisé en ce**
**que** l'agencement de bagues d'étanchéité (7) présente, sur les côtés des corps d'électrode (2.1, 2.1') tournés l'un vers l'autre, portant les zones de contact (2.2, 2.2'), chaque fois au moins un bourrelet d'étanchéité formé dessus (fig. 1A) qui sont mis en coïncidence l'un avec l'autre par rapport aux deux corps d'électrode (2.1, 2.1') et reliés entre eux par le moyen de liaison (7.1) ou
**qu'**au moins une bague d'étanchéité qui fait le tour des zones de contact (2.2, 2.2') est disposée entre les côtés tournés l'un vers l'autre des deux corps d'électrode (2.1, 2.1'), laquelle est reliée sur ses deux côtés tournés vers les corps d'électrode (2, 2') chaque fois par des moyens de liaison (7.1) aux corps d'électrode (2, 2').

4. Paquet d'électrodes selon la revendication 2 ou 3,
**caractérisé en ce**
**qu'**au moins une couche intermédiaire (6) est disposée entre les zones de contact (2.2, 2.2') tournées l'une vers l'autre.

5. Paquet d'électrodes selon la revendication 1 ou 2,
**caractérisé en ce**
**que** l'agencement de bagues d'étanchéité (7) fait partie d'une feuille recouvrant la zone de contact (2.2, 2.2'), qui forme au moins une couche intermédiaire isolante (6) entre les zones de contact (2.2, 2.2') tournées l'une vers l'autre, une partie respective étant reliée par un moyen de liaison (7.1) respectif à un corps d'électrode (2.1, 2.1') qui lui est associé.

6. Paquet d'électrodes selon une des revendications précédentes,
**caractérisé en ce**
**qu'**au moins une des deux électrodes (2, 2') est munie d'une languette à tirer (4) pour séparer les deux électrodes (2,2').

7. Paquet d'électrodes selon la revendication 6,
**caractérisé en ce**
**que** ladite au moins une languette à tirer (4) est attachée à l'agencement de bagues d'étanchéité (7) ou à ladite au moins une couche intermédiaire (6).

8. Paquet d'électrodes selon une des revendications 4 à 7,
**caractérisé en ce**
**que** la couche intermédiaire (6) est intégrée en tant que moyen d'action pour un test électrique des électrodes.
